(19) Europäisches Patentamt
European Patent Office
Office européen des brevets

(11) **EP 2 389 208 B1**

(12) **FASCICULE DE BREVET EUROPEEN**

(45) Date de publication et mention
de la délivrance du brevet:
**29.04.2020 Bulletin 2020/18**

(21) Numéro de dépôt: **09801743.7**

(22) Date de dépôt: **04.12.2009**

(51) Int Cl.:
*A61M 5/142* (2006.01)     *A61K 9/00* (2006.01)
*A61M 5/172* (2006.01)

(86) Numéro de dépôt international:
**PCT/FR2009/052412**

(87) Numéro de publication internationale:
**WO 2010/081947 (22.07.2010 Gazette 2010/29)**

(54) **SYSTÈME DE PILOTAGE DE MOYENS D'INJECTION D'AGENTS D'ANESTHÉSIE OU DE SÉDATION EN VUE DE L'INDUCTION DE CELLE-CI**

SYSTEM ZUR KONTROLLE VON MITTELN ZUR INJEKTION VON NARKOSE- ODER BERUHIGUNGSMITTELN ZUR INDUKTION VON ANÄSTHESIE ODER SEDIERUNG

SYSTEM FOR CONTROLLING MEANS FOR INJECTION OF ANAESTHETICS OR SEDATIVES WITH A VIEW TO INDUCING ANAESTHESIA OR SEDATION

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **15.01.2009 FR 0950213**

(43) Date de publication de la demande:
**30.11.2011 Bulletin 2011/48**

(73) Titulaire: **MedSTEER**
**95410 Groslay (FR)**

(72) Inventeurs:
• **CHAZOT, Thierry**
**F-95410 Groslay (FR)**
• **LIU, Ngai**
**F-75014 Paris (FR)**
• **TRILLAT, Bernard**
**F-75009 Paris (FR)**

(74) Mandataire: **Lavoix**
**2, place d'Estienne d'Orves**
**75441 Paris Cedex 09 (FR)**

(56) Documents cités:
**EP-A- 1 547 631      WO-A-2008/059289
WO-A-2008/086624      US-A1- 2003 051 737
US-A1- 2004 079 372   US-A1- 2007 010 756**

**Description**

La présente invention concerne un système de pilotage de moyens d'injection d'agents d'anesthésie ou de sédation en mode d'anesthésie ou de sédation intraveineuse à objectif de concentration ou de débit massique, à un patient, en vue de l'induction et de l'entretien de cette anesthésie ou de cette sédation.

Une anesthésie générale pour un acte chirurgical, peut se définir comme un état réversible, dans lequel le patient doit être inconscient grâce à l'utilisation d'un agent hypnotique, analgésié grâce à l'utilisation d'un agent morphinomimétique et dont les muscles doivent être relâchés grâce à l'utilisation de curares qui facilitent l'acte chirurgical.

Cette anesthésie générale est alors obtenue par inhalation de gaz ou par injection intraveineuse d'agents d'anesthésie ou par leur association.

L'utilisation exclusive d'agents d'anesthésie intraveineux est une pratique habituelle. L'évaluation de la profondeur de l'anesthésie ou la titration des agents d'anesthésie se fait à l'aide de critères cliniques ou paracliniques.

Actuellement, l'adaptation posologique des agents d'anesthésie est réalisée par rapport aux modifications somatiques (mouvements) ou du système nerveux autonome (tachycardie, hypertension, sudation, modifications pupillaires) qui sont provoquées par la chirurgie ou qui témoignent d'un sous dosage médicamenteux.

Cependant, les signes cliniques ne sont pas toujours spécifiques ou peuvent être absents. Les mouvements ne sont plus un critère fiable lorsqu'on utilise des curares.

Les modifications cardiovasculaires ne sont pas spécifiques des agents d'anesthésie utilisés et peuvent être liées à la chirurgie (saignement, clampage vasculaire...) ou au patient (hypertension artérielle, traitements cardiovasculaires...).

L'anesthésie intraveineuse à objectif de concentration également appelée AIVOC, est une méthode pratiquée pour titrer l'injection d'agent hypnotique ou d'agent morphinomimétique.

Cette méthode consiste à utiliser un modèle pharmacocinétique de l'agent qui calcule une concentration plasmatique et/ou une concentration au « site effet » c'est-à-dire au niveau cérébral, en faisant l'hypothèse qu'il existe une relation entre la concentration calculée et l'effet du médicament.

Mais, les concentrations calculées par les modèles ont une très mauvaise corrélation avec l'état clinique du patient et leur utilisation n'a pas montré d'amélioration de la prise en charge des opérés par rapport à une utilisation standard (c'est-à-dire en perfusion massique) des mêmes agents anesthésiques.

L'intérêt des outils d'AIVOC est d'offrir aux médecins la possibilité d'adaptations posologiques plus rapides que des variations exprimées en concentrations massiques.

La présence d'un anesthésiologiste reste donc indispensable lors d'une anesthésie.

Cependant, un anesthésiologiste n'est pas toujours disponible lors de situations d'urgence, de conflits militaires ou lorsque le patient ne peut pas être déplacé.

Un moyen pour mesurer la profondeur de l'anesthésie ou l'effet des agents d'anesthésie est de mesurer l'activité électrocorticale ou l'électroencéphalogramme (EEG) du patient.

Les agents d'anesthésie modifient la morphologie du signal EEG de manière spécifique à chaque agent. Cependant, seuls des électroencéphalographistes entraînés peuvent détecter ces modifications. La mesure de l'EEG a surtout été utilisée en anesthésie lors de protocoles de recherche pour quantifier l'effet des agents d'anesthésie.

L'interprétation en temps réel des modifications de l'EEG en bloc opératoire a été facilitée par l'apparition de moniteurs qui permettent une analyse en temps réel de ce signal d'EEG. Ces moniteurs calculent différents paramètres à partir de l'analyse spectrale de l'EEG et les combinent pour fournir un signal ou index de profondeur d'anesthésie.

Le moniteur BIS de la société Aspect Medical system Inc., est utilisé pour mesurer la profondeur de l'anesthésie en calculant un index à partir d'une analyse bispectrale de l'EEG. Cet index varie de 0 à 100, 0 représentant un tracé isoélectrique ou plat et 100 représentant un tracé d'un patient éveillé. Lors d'une anesthésie, la recommandation est de maintenir cet index dans un intervalle entre 45 et 60 pour obtenir des conditions satisfaisantes à la réalisation d'un acte chirurgical. Un tel moniteur permet de mesurer la profondeur de l'anesthésie et de titrer l'agent hypnotique.

En effet, il a déjà été proposé d'utiliser cet index BIS pour administrer en boucle fermée un agent hypnotique intraveineux lors de l'entretien de l'anesthésie (voir par exemple le document US 7,220,240), ou lors de l'induction et de l'entretien d'une anesthésie générale.

Il existe dans l'état de la technique un autre moniteur tel que par exemple le moniteur d'Entropy de la société Datex-Ohmeda Inc. Ce moniteur quantifie le désordre, c'est-à-dire l'entropie, dans le signal EEG, ce signal pour un patient anesthésié étant caractérisé par des ondes amples, synchronisées et ordonnées avec peu de désordre et donc une entropie faible.

Il suffit alors de maintenir cet index dans l'intervalle 40 à 60 pour obtenir des conditions satisfaisantes à la réalisation de la chirurgie. Ce moniteur fournit deux informations, la première étant appelée l'information de «State Entropy (SE)» qui mesure la profondeur de l'hypnose et la deuxième étant appelée la « Response Entropy (RE)» qui mesure le déficit en antinociception. Ce moniteur a déjà été proposé pour la perfusion automatisée d'un agent hypnotique (voir par exemple le document US 6,631,291).

D'autres documents proposent la mise en œuvre de systèmes à boucle fermée comme par exemple le document US 2004/079372 ou de systèmes d'aide proposant aux opérateurs des informations permettant de les guider lors des opérations comme par exemple le document EP 1547631.

Cependant aucun de ces systèmes n'a donné pleinement satisfaction.

Le but de l'invention est d'optimiser l'utilisation de ces systèmes.

A cet effet, l'invention a pour objet un système de pilotage de moyens d'injection d'agents d'anesthésie ou de sédation en mode d'anesthésie ou de sédation intraveineuse à objectif de concentration ou de débit massique, à un patient, en vue de l'induction et de l'entretien de cette anesthésie ou de cette sédation, les moyens d'injection d'agents d'anesthésie comprenant des premiers moyens d'injection d'un agent hypnotique et des seconds moyens d'injection d'un agent morphinomimétique, comportant :

- des moyens d'acquisition d'un signal représentatif de l'activité électrocorticale du patient,
- des moyens d'analyse de ce signal pour en tirer un signal de profondeur d'anesthésie, et
- des moyens de suivi de la valeur et de l'évolution dans le temps de ce signal de profondeur d'anesthésie ;

caractérisé en ce que les moyens de suivi de la valeur et de l'évolution dans le temps du signal de profondeur d'anesthésie sont associés à des moyens de calcul d'ordres de pilotage des premiers et des seconds moyens d'injection des agents hypnotique et morphinomimétique, afin d'amener automatiquement le signal de profondeur d'anesthésie au-dessous d'une valeur cible pendant au moins 30 secondes lors d'une phase d'induction de l'anesthésie, la phase d'induction de l'anesthésie étant la période entre le début de l'administration des agents et le maintien du signal de profondeur d'anesthésie au moins 30 secondes sous la valeur cible, puis de réguler automatiquement celui-ci en boucle fermée dans une plage prédéterminée lors de l'entretien de cette anesthésie ; et en ce que les moyens de calcul des ordres de pilotage des moyens d'injection comprennent :

- des moyens d'entrée par un opérateur d'un type d'induction d'anesthésie choisi parmi un ensemble de types d'induction se différenciant par la concentration initiale d'agent hypnotique, comprenant des moyens d'établissement de la concentration initiale d'agent hypnotique, à laquelle est associée une concentration initiale d'agent morphinomimétique prédéterminée selon le type d'induction sélectionné, et
- des moyens d'établissement de valeurs de signal de profondeur d'anesthésie intermédiaires pour définir des paliers lors de la phase d'induction de l'anesthésie.

Selon d'autres aspects, le système selon l'invention présente l'une ou plusieurs des caractéristiques suivantes :

- les moyens d'établissement sont adaptés pour établir trois valeurs intermédiaires de paliers d'anesthésie ;
- les premiers moyens d'injection de l'agent hypnotique reçoivent des ordres de pilotage à une première fréquence et en ce que les seconds moyens d'injection de l'agent morphinomimétique reçoivent des ordres de pilotage à une seconde fréquence différente de la première ;
- la seconde fréquence est supérieure à la première fréquence ;
- il comporte des moyens d'entrée par un opérateur de la valeur cible de signal de profondeur d'anesthésie et des moyens d'établissement d'une valeur cible par défaut,
- les moyens de calcul comportent des moyens de limitation et/ou d'inhibition d'ordres de pilotage en sens opposés des premiers et seconds moyens d'injection,
- les moyens de calcul sont adaptés pour augmenter la concentration d'agent hypnotique après un nombre prédéterminé de modifications de la concentration d'agent morphinomimétique pour ramener le signal de profondeur d'anesthésie dans la plage prédéterminée autour de la valeur cible,
- le nombre prédéterminé de modifications est égal à trois,
- les moyens de calcul sont adaptés pour calculer l'écart entre la valeur courante du signal de profondeur d'anesthésie et la valeur cible, et pour comparer cet écart à des valeurs de seuil prédéterminées pour, lorsque la valeur courante du signal de profondeur d'anesthésie est au-dessus de la valeur cible :

  • lorsque l'écart est inférieur à un premier seuil, agir prioritairement sur la concentration d'agent morphinomimétique en l'augmentant,
  • lorsque l'écart est supérieur au premier seuil et inférieur à un second seuil, augmenter la concentration d'agent morphinomimétique et augmenter celle de l'agent hypnotique dans une faible proportion, et
  • lorsque l'écart est supérieur au second seuil, augmenter la concentration d'agent morphinomimétique et celle de l'agent hypnotique dans une plus forte proportion,

  pour ramener la valeur du signal de profondeur d'anesthésie dans la plage prédéterminée,

- les moyens de calcul sont adaptés pour calculer l'écart entre la valeur courante du signal de profondeur d'anesthésie et la valeur cible, et pour comparer cet écart à des valeurs de seuil prédéterminées si le signal est inférieur à la valeur cible pour, lorsque la valeur courante du signal de profondeur d'anesthésie est en dessous de la valeur cible :

- • lorsque l'écart est inférieur à un troisième seuil, agir prioritairement sur la concentration d'agent morphinomimétique en la réduisant,
- • lorsque l'écart est inférieur au troisième seuil et supérieur à un cinquième seuil, réduire la concentration d'agent morphinomimétique et diminuer dans une faible proportion celle de l'agent hypnotique, et
- • lorsque l'écart est inférieur au quatrième seuil, réduire la concentration d'agent morphinomimétique et celle de l'agent hypnotique dans une plus forte proportion,

pour ramener la valeur du signal de profondeur d'anesthésie dans la plage prédéterminée,
- il comporte des moyens d'établissement de valeurs limites hautes et basses de concentrations des agents hypnotique et morphinomimétique,
- il comporte des moyens d'entrée manuelle par l'opérateur des valeurs limites hautes et basses de concentrations des agents hypnotique et morphinomimétique,
- il comporte des moyens de désactivation de l'un et/ou l'autre des moyens de calcul des ordres de pilotage des moyens d'injection, afin de permettre à l'opérateur une reprise manuelle de la commande des moyens d'injection des agents,
- il comporte des moyens de maintien des ordres de commande à leur dernière valeur avant une perte du signal d'activité électrocorticale,
- il comporte des moyens d'alerte sonore et/ou visuelle de l'opérateur en cas de perte du signal d'activité électrocorticale, et
- les moyens d'injection comprennent des moyens motorisés associés à des contrôleurs de pilotage de leur fonctionnement.

L'invention a aussi pour objet un système de pilotage selon la revendication 1.

Selon d'autres aspects, le système selon l'invention présente l'une ou plusieurs des caractéristiques des revendications 2 à 16.

L'invention sera mieux comprise à l'aide de la description qui va suivre donnée uniquement à titre d'exemple et faite en se référant aux dessins annexés sur lesquels :

- la figure 1 représente un schéma synoptique de la structure et du fonctionnement d'un système de pilotage selon l'invention, et
- la figure 2 représente un tableau d'étapes d'induction d'une anesthésie.

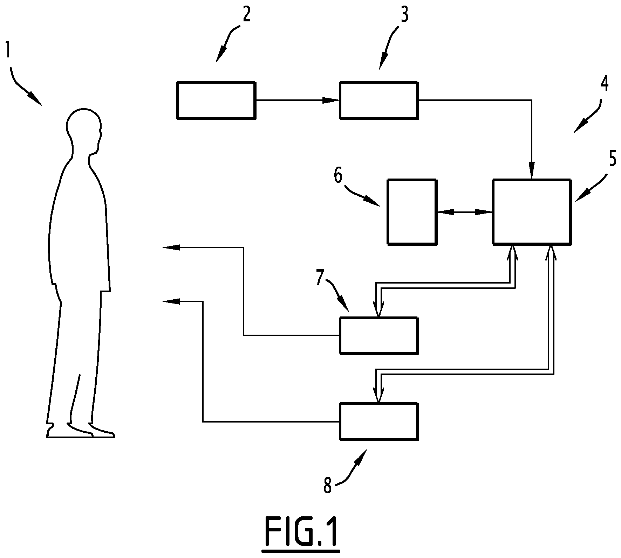

On a en effet illustré sur cette figure 1, un système de pilotage de moyens d'injection d'agents d'anesthésie ou de sédation en mode d'anesthésie ou de sédation intraveineuse à objectif de concentration ou de débit massique, à un patient, en vue de l'induction et de l'entretien de cette anesthésie ou de cette sédation.

Sur cette figure, le patient est désigné par la référence générale 1 et le système comporte alors des moyens d'acquisition d'un signal représentatif de l'activité électrocorticale du patient, ces moyens étant désignés par la référence générale 2 sur cette figure.

La sortie de ces moyens d'acquisition est raccordée à des moyens d'analyse du signal correspondant pour en tirer un signal de profondeur d'anesthésie, ces moyens d'analyse étant désignés par la référence générale 3.

A titre d'exemple, ces moyens d'analyse peuvent par exemple délivrer un signal sous la forme d'un index BIS ou d'entropie, comme cela a été indiqué précédemment.

La sortie de ces moyens d'analyse est raccordée à des moyens de suivi de la valeur et de l'évolution dans le temps de ce signal de profondeur d'anesthésie, associés à des moyens de calcul d'ordres de pilotage de moyens d'injection, permettant de réguler automatiquement en boucle fermée, le signal de profondeur d'anesthésie dans une plage prédéterminée autour d'une valeur cible.

Ces moyens sont désignés par la référence générale 4 sur cette figure et comprennent par exemple tout calculateur 5 approprié, associé à des moyens de stockage d'informations, désignés par la référence 6.

En fait, les moyens d'injection d'agents d'anesthésie comprennent des premiers moyens d'injection d'un agent hypnotique recevant des ordres de pilotage à une première fréquence, ces moyens d'injection étant désignés par la référence générale 7 sur cette figure et des seconds moyens d'injection d'un agent morphinomimétique recevant des ordres de pilotage à une seconde fréquence, différente de la première fréquence, ces moyens étant désignés par la référence générale 8 sur cette figure. De plus les premiers moyens d'injection d'un agent hypnotique 7 et les seconds moyens d'injection d'un agent morphinomimétique 8 renvoient une information sur la quantité d'agent administré, respectivement de l'agent hypnotique et de l'agent morphinomimétique, et un contrôleur vérifie régulièrement que les ordres sont exécutés.

On notera que la seconde fréquence est supérieure à la première fréquence dans le mode de réalisation illustré.

Ainsi, le système de pilotage selon l'invention permet de piloter l'injection des agents hypnotique et morphinomimétique, à partir du même signal d'index BIS ou d'entropie pour induire et entretenir l'anesthésie ou la sédation en maintenant un niveau de profondeur d'anesthésie stable.

Ceci est alors réalisé en utilisant un contrôleur de type CLASS (pour « Closed-Loop Anesthesia Safety System ») qui pilote les moyens d'injection en mode AIVOC, en utilisant l'information qui provient du moniteur après analyse de l'activité EEG du patient.

Les informations délivrées par le moniteur comprennent :

- l'index de qualité du signal,
- la valeur absolue de l'index enregistrée par exemple toutes les cinq secondes,
- la valeur de l'EMG (pour « Electromyogramme ») ou la RE (« Response Entropy »),
- la présence et la quantification de BSR (pour « Burst Suppression Ratio »),
- la pente de variation de l'index.

Le calculateur mesure et calcule alors à partir de cet index, en utilisant des fenêtres temporelles successives :

- l'évolution des oscillations en fréquence et en amplitude,
- le pourcentage de temps compris dans l'intervalle 40-60,
- le pourcentage de temps de valeurs inférieures à 45,
- le pourcentage de temps de valeurs supérieures à 55.

Le contrôleur CLASS est du type contrôleur/régulateur Proportionnel Intégral Dérivé (PID), c'est-à-dire un organe de contrôle permettant d'effectuer une régulation en boucle fermée d'un système industriel. Les régulateurs PID sont les plus utilisés dans les systèmes d'asservissement. Le PID permet trois actions simultanées sur l'erreur entre l'objectif (ou consigne) et la mesure :

- une action proportionnelle : l'erreur est multipliée par un gain $K_c$ (gain du contrôleur),
- une action intégrale : l'erreur est intégrée sur un intervalle de temps $T_i$, appelé temps intégral, et
- une action dérivée : l'erreur est dérivée suivant un temps $T_d$, appelé temps dérivé.

Si le temps est une variable continue, le contrôleur PID est décrit par l'équation (1) suivante :

$$u(t) = K_c e(t) + \frac{K_c}{T_i} e(t) \int_0^t e(t) dt + K_c T_d + K_c T_d \frac{de(t)}{dt} \quad (1)$$

Quand le temps devient une variable discrète et numérisée (par exemple si la concentration est maintenue constante entre deux mesures du BIS), le PID peut s'écrire suivant l'équation (2) :

$$u(t) = u(t - T_s) + K_c [e(t) - e(t - T_s)] + \frac{K_c T_s}{T_i} e(t) + \frac{K_c T_d}{T_s} [e(t) - 2e(t - T_s) + e(t - 2T_s)] \quad (2)$$

où $T_s$ représente l'intervalle de temps entre les mesures pour un tel contrôleur PID numérisé.

Dans ces deux équations (1) et (2), $u(t)$ est la sortie du contrôleur et $e(t) = y_{sp} - y(t)$ est l'Index Error où $y_{sp}$ est l'objectif (ou consigne, connu sous le nom anglais, « setpoint »).

Le contrôleur modifie la concentration cible au site effet, notée Ce, d'agent hypnotique tel que le Propofol ou d'agent morphinomimétique tel que le Remifentanil pour maintenir le BIS à un objectif de 50, par exemple. L'algorithme peut se décomposer en deux termes : un terme d'amplification de la correction (AFB) et un terme de rétroaction (Feed-forward).

Le contrôleur CLASS a une structure en cascade qui intègre les éléments suivants :

1/ l'Index Error

L'Index Error est calculé toutes les cinq secondes par exemple et représente la différence entre l'index mesuré et l'index nominal, c'est-à-dire celui souhaité. Cet index nominal peut être entré par un opérateur, et représente alors la valeur cible du signal de profondeur d'anesthésie. Il peut également être établi par des moyens d'établissement d'une valeur cible par défaut, par exemple à 50. Si l'Index Error est différent de zéro, une modification de concentration des agents est réalisée après un temps d'attente prédéterminé.

2/ Délai entre chaque modification

Un délai ou une période réfractaire entre chaque modification doit être respecté. Ce délai est calculé initialement grâce au modèle pharmacocinétique de chaque agent d'anesthésie. Par exemple le modèle de Schnider peut être choisi pour l'agent hypnotique et le modèle de Minto pour l'agent morphinomimétique. Le délai entre chaque modification de concentration dépend alors du temps nécessaire pour atteindre la concentration au site effet calculé par le modèle. Entre chaque modification, le contrôleur attend le temps nécessaire pour atteindre la concentration calculée au site effet. A ce temps théorique, un délai supplémentaire variable, qui dépend de la dernière modification de concentration, est ajouté. Lorsque les modifications de concentration sont faibles, le délai d'équilibre ou de stabilisation au site effet est plus long et on ajoute un délai supplémentaire variant de cinq secondes à deux minutes par exemple.

Dans le cas de l'agent morphinomimétique, chaque ordre de baisse de la valeur cible induit une augmentation du délai supplémentaire et une diminution du seuil de déclenchement pour une nouvelle baisse. Le premier ordre de hausse de la valeur cible réinitialise le délai supplémentaire et le seuil de déclenchement des baisses de la valeur cible.

Par contre, si la rétroaction (« Feed-forward », en anglais) est activée, celle-ci est prioritaire et une modification de concentration peut être déclenchée automatiquement et immédiatement.

3/ Amplification de la correction (AFB pour « Amplification of the Feedback », en anglais)

L'amplification de la correction est le gain qui permet la modification de la concentration. Cette correction est possible si l'index de qualité du signal est supérieur à 50 %. La correction dépend de l'Index Error, de l'activité EMG, de l'agent anesthésique et de la rétroaction. L'AFB modifie la concentration selon la formule suivante :

(nouvelle concentration) = (concentration actuelle) x (1 + Index Error/AFB)

La correction peut s'exprimer comme :

$$C_e(t) = C_e(t - T_s)\left[1 - \frac{e(t)}{AFB}\right]$$

où :

- $-e(t)$ est l'Index Error, avec $e(t) = BIS_{sp} - BIS(t)$, et
- $T_S$ est le temps minimal entre deux périodes de modification de la concentration. Il est égal au minimum au temps nécessaire pour atteindre le pic de l'effet du médicament. Ce délai est ajusté de 5 à 120 secondes en fonction de la dernière correction. Une précédente correction de faible amplitude augmente le délai de la modification suivante.

Si on remplace dans l'équation précédente $u(t) = C_e(t)$, $y(t) = BIS(t)$ et $y_{sp} = BIS_{sp}$, la correction peut s'écrire :

$$u(t) = u(t - T_s)\left[1 - \frac{e(t)}{AFB}\right] \quad (3)$$

La comparaison des équations (2) et (3) permet alors de remarquer que le contrôleur de la correction est une fonction intégrale et que le gain est une fonction du type $u(t - T_S)$ :

$$\frac{K_c T_d}{T_i} = \frac{C_e(t - T_s)}{AFB} \quad (4)$$

L'augmentation de concentration des agents d'anesthésie est d'autant plus importante que l'Index Error est important. Cependant les variations de concentration sont limitées lors des phases de décroissance de celles-ci afin d'éviter des périodes réfractaires supérieures par exemple à trois minutes. L'AFB est spécifique à chaque agent, à la phase d'anesthésie (induction ou entretien), à chaque Index Error et aux concentrations en cours des agents. La détermination de l'AFB dépend du signe et de l'importance de l'Index Error.

4/ Rétroaction (« Feed-forward », en anglais)

Cette fonction permet d'amplifier la correction de la concentration. Dans la hiérarchie du contrôleur CLASS, le Feed-forward est prioritaire et peut intervenir n'importe quand.

Cette fonction est inhibée si l'index de qualité du signal est inférieur à 50, si le pourcentage moyen des BSR (selon l'acronyme anglais « Burst Suppression Ratio ») est supérieure à 5 % lors des quatre dernières minutes, et si l'activité EMG est supérieure à 47 dB, que l'on considère alors comme de l'artefact, si une amplification de correction est en cours et si on est en phase d'induction de l'anesthésie.

Cette fonction est activée lors de la phase d'entretien de l'anesthésie, si l'index mesuré est supérieur à 60 ou si l'Index Error ($-e(t)$) est supérieur à 10, si la pente de l'index augmente de plus de 10 points en moins de quinze secondes par exemple, ou si l'activité EMG dépasse 41 dB pour l'agent hypnotique tel que le Propofol et 37 dB pour l'agent morphinomimétique tel que le Remifentanil. Enfin, cette fonction peut être activée par des oscillations de l'index ou de l'activité EMG.

La condition peut alors s'exprimer comme $e(t) - 2e(t-1) + e(t-2) > 10$ avec une mesure toutes les cinq secondes.

Lorsque le Feed-forward est activé, une correction de concentration est faite immédiatement avec un AFB proportionnel à l'Index Error et à la concentration des agents en cours: $AFB = -e(t).EMG.u(t - T_S)$ où

$$u(t) = u(t - T_S)\left[1 - \frac{e(t)}{AFB}\right] = u(t - T_S)\left[1 + \frac{1}{EMG.u(t - T_S)}\right],$$ ce qui donne :

$$u(t) = u(t - T_S) + \frac{1}{EMG}$$

De plus, si la concentration en cours est basse, le contrôleur détermine une concentration par défaut afin d'éviter des modifications trop faibles. Par exemple :

- si la concentration d'agent hypnotique en cours est inférieure à 1,3 µg/mL, l'activation de cette fonction provoque une augmentation minimum de concentration à 1,8 µg/mL de la valeur cible au site effet, et
- si la concentration d'agent morphinomimétique en cours est inférieure à 4 µg/mL, l'activation de la fonction provoque au minimum une augmentation à 4 µg/mL de la valeur cible au site effet.

5/ Règles d'interaction entre les agents hypnotique et morphinomimétique

Dans le système selon l'invention, il y a donc deux contrôleurs, l'un pour l'agent hypnotique et l'autre pour l'agent morphinomimétique. Ces deux contrôleurs sont activés en même temps lors de l'induction de l'anesthésie, puis ils fonctionnent de manière indépendante.

Comme les propriétés pharmacocinétiques d'un agent morphinométique, de type Remifentanil par exemple, sont différentes de celles d'un agent hypnotique, de type Propofol par exemple, le délai entre chaque modification est plus court, les délais d'attente ajoutés sont plus courts et les conditions de déclenchements plus resserrées autour de la valeur d'index cible pour l'agent morphinomimétique par rapport à ceux pour l'agent hypnotique. Il en résulte que les modifications pour l'agent morphinomimétique sont en général plus fréquentes que pour l'agent hypnotique en l'absence d'activation de la fonction Rétroaction.

Par ailleurs, des règles d'interaction sont activées pour limiter voire éviter des décisions et des ordres de pilotage en sens opposés des premiers et seconds moyens d'injection. Ainsi, lorsque la concentration d'agent morphinomimétique augmente, le contrôleur limite la possibilité à la concentration d'agent hypnotique de diminuer. De même, lorsque la concentration d'agent morphinomimétique diminue, le contrôleur limite la possibilité à la concentration d'agent hypnotique d'augmenter. Lorsque c'est la concentration d'agent hypnotique qui augmente, le contrôleur limite la possibilité à la concentration d'agent morphinomimétique de diminuer. De même, si la concentration d'agent hypnotique diminue, le contrôleur limite la possibilité à la concentration d'agent morphinomimétique d'augmenter.

Des variations de grande amplitude ou répétées d'une concentration d'un des agents conduisent à interdire des variations inverses de concentration de l'autre agent en fixant transitoirement la limite inférieure de la concentration de cet agent à la valeur courante.

Par ailleurs, s'il y a plus de trois modifications positives (augmentations) successives par exemple de la concentration d'agent morphinomimétique, une correction positive de la concentration d'agent hypnotique est effectuée. Bien entendu, un nombre différent de modifications successives peut être envisagé.

6/ Administration des agents en mode AIVOC

L'administration des agents d'anesthésie en mode d'Anesthésie IntraVeineuse à Objectif de Concentration (AIVOC) est une méthode utilisée en pratique clinique depuis des années et qui met en œuvre une pompe de perfusion associée à un microprocesseur. Le programme du microprocesseur contient alors le modèle pharmacocinétique qui modélise l'élimination et le métabolisme du médicament.

En donnant l'âge, le sexe, le poids, la taille du patient et la concentration plasmatique souhaitée, le programme intégré calcule et administre un bolus de médicament nécessaire pour obtenir la concentration souhaitée.

Il permet également de garder une concentration théorique calculée stable.

Le programme permet aussi de calculer le temps théorique nécessaire pour obtenir une nouvelle concentration plasmatique au site effet.

Si l'index de qualité du signal est inférieur à 50 %, l'outil maintient la dernière concentration choisie. Le système permet alors l'affichage en temps réel du calcul des concentrations théoriques plasmatiques et au site effet, la concentration choisie par l'outil, les courbes de concentrations, le délai de la prochaine modification le cas échéant et le débit massique. Ceci permet alors de calculer le temps nécessaire pour atteindre l'équilibre de la concentration cible choisie, évalué par le modèle pharmacocinétique utilisé.

Dans le système selon l'invention, il y a en fait deux contrôleurs de type PID qui ont la même structure et qui pilotent l'un la concentration d'agent hypnotique et l'autre celle d'agent morphinomimétique, simultanément à partir d'informations qui proviennent du même signal d'index. En l'absence d'activation du Feed-forward ou des règles d'interaction et s'il a respecté le délai d'attente entre deux modifications, le contrôleur peut décider d'une modification de concentration.

Comme le délai d'action donné par le modèle pharmacocinétique est plus rapide pour l'agent morphinomimétique tel que le Rémifentanil, les concentrations de cet agent sont modifiées plus fréquemment que celles de l'agent hypnotique tel que le Propofol. Cette modification dépend du signe de l'Index Error. Un Index Error positif provoque une augmentation de concentration et un Index Error négatif provoque une diminution de concentration.

Si l'Index Error est inférieur à ± 2 pour l'agent morphinomimétique et à ± 3 pour l'agent hypnotique, il n'y a pas de modification. Au-delà des valeurs indiquées d'Index Error, le contrôleur fait une modification. A chaque valeur d'Index Error sont attribués un AFB et un délai spécifique pour les deux agents.

Le concept de la gestion automatique de l'agent morphinomimétique repose sur le fait que des variations d'index faibles préfigurent des variations plus importantes et sont imputables à un défaut d'analgésie ponctuel plus qu'à un déficit d'agent hypnotique. Les paramètres de réglages ont donc été établis pour que la réactivité du système morphinomimétique soit plus importante que celle du système hypnotique. Le nombre de changements de concentration d'agent morphinomimétique est donc plus important que celui de l'agent hypnotique en raison d'une période réfractaire plus courte entre chaque modification et un seuil de déclenchement plus bas. Le comportement de la boucle fermée de l'agent hypnotique est alors d'éviter d'avoir un index en dessous de 40 ou au dessus de 60.

Le contrôleur CLASS enregistre alors les informations provenant du moniteur et des moyens d'injection qui peuvent par exemple se présenter sous la forme de moyens motorisés tels que des seringues électriques.

Comme cela a été indiqué précédemment, les informations à fournir lors de la mise en route du système sont l'âge, la taille, le poids, le sexe du patient et l'anesthésiologiste décide de la première concentration d'agent hypnotique. Le système calcule alors la première concentration de l'agent morphinomimétique. Ainsi, ce système permet de maintenir l'index par exemple dans un intervalle de 40 à 60.

L'algorithme intégré dans les contrôleurs CLASS comporte deux parties spécifiques, l'une pour la phase d'induction de l'anesthésie et l'autre pour la phase d'entretien de celle-ci. Le passage entre les deux phases se fait de façon automatique.

Le système selon l'invention permet de réaliser automatiquement la phase d'induction, qui se définit comme la période entre le début de l'administration des agents et le maintien du signal EEG au moins 30 secondes sous la valeur 60 par exemple.

La seule décision de l'utilisateur est alors de choisir la première concentration d'agent hypnotique comme le Propofol entre 1 et 5 µg/mL. En fait, l'utilisateur choisit ainsi un type d'induction d'anesthésie parmi un ensemble de types d'induction qui se différencient par la concentration initiale d'agent hypnotique. Le contrôleur détermine alors automatiquement la première concentration d'agent morphinomimétique tel que le Rémifentanil en rapport avec la concentration en agent hypnotique demandée par l'utilisateur, et prédéterminée selon le type d'induction sélectionné.

Par exemple, quatre types d'induction différents peuvent être envisagés : les inductions de type A, de type B, de type C, et de type D. Les différents types d'induction se distinguent par :

- la valeur de la première concentration cible d'agent morphinomimétique tel que le Rémifentanil,
- les valeurs de délais d'attente après atteinte de l'équilibre de concentration cible de chaque agent, et
- les seuils de valeurs d'index qui déterminent le changement de palier.

Le principe de l'induction ainsi conçue repose sur l'idée que le médecin en charge du patient dispose des moyens d'évaluation du risque lié à un surdosage d'agents anesthésiques lors de l'induction. Il est de bon sens que plus le risque est élevé, moins la valeur de la concentration cible de départ doit être haute. En conséquence, la première valeur de concentration cible d'agent morphinomimétique tel que le Rémifentanil, est indexée à la première valeur de concentration cible d'agent hypnotique tel que le Propofol.

L'induction de type A peut être utilisée pour les patients ne présentant pas de problèmes majeurs et dont un surdosage modéré d'hypnotique ne pose pas de problème. C'est une séquence rapide d'induction qui est demandée. La concentration d'agent hypnotique tel que le Propofol est supérieure à 2,8 µg/mL, la première concentration d'agent morphinomimétique tel que le Rémifentanil est alors de l'ordre de 5 ng/mL.

L'induction de type B peut être utilisée pour des patients présentant des problèmes mineurs dont on souhaite éviter un surdosage pouvant entraîner une hypotension artérielle. Dans ce cas, la concentration d'agent hypnotique tel que le Propofol, demandée, peut être comprise entre 2,4 et 2,8 µg/mL, la première concentration d'agent morphinomimétique tel que le Rémifentanil étant de l'ordre de 5 ng/mL.

L'induction de type C peut être utilisée pour des patients fragiles pour lesquels on cherche à obtenir une induction avec peu de modification hémodynamique. C'est une séquence d'induction lente. Dans ce cas, la concentration demandée d'agent hypnotique tel que le Propofol est comprise entre 2,1 et 2,4 µg/mL, la première concentration d'agent morphinomimétique tel que le Rémifentanil étant alors de l'ordre de 4 ng/mL.

L'induction de type D peut être utilisée pour des patients particulièrement fragiles ou lors de la prise en charge de patients de réanimation déjà sédatés. Ce type d'induction est sélectionné par une première concentration cible d'agent hypnotique tel que le Propofol inférieure ou égale à 2,1 µg/mL.

Par ailleurs, le système comporte également des moyens d'établissement de valeurs de signal de profondeur d'anesthésie intermédiaires pour définir des paliers lors de la phase d'induction par exemple pour les quatre types d'induction.

Le système utilise alors comme signal entrant l'index de qualité du signal, la valeur absolue de l'index enregistrée par exemple toutes les cinq secondes, la valeur de l'EMG et l'Index Error.

Selon le type d'induction et le palier, les valeurs de l'AFB, du délai et l'index nominal sont spécifiques.

Par exemple, trois valeurs intermédiaires de paliers d'anesthésie peuvent être envisagées.

L'objectif du système est alors d'établir un premier palier avec un index nominal égal à 80 par exemple. L'index mesuré est alors pondéré en fonction de l'activité EMG et on diminue la valeur de l'index si cette activité EMG est élevée et selon la valeur de l'index mesurée. Les délais d'attente avant une nouvelle modification sont alors fixés. Ces délais varient de 15 à 120 secondes mais un délai maximum entre deux variations de concentration cible ou par palier d'induction est fixé.

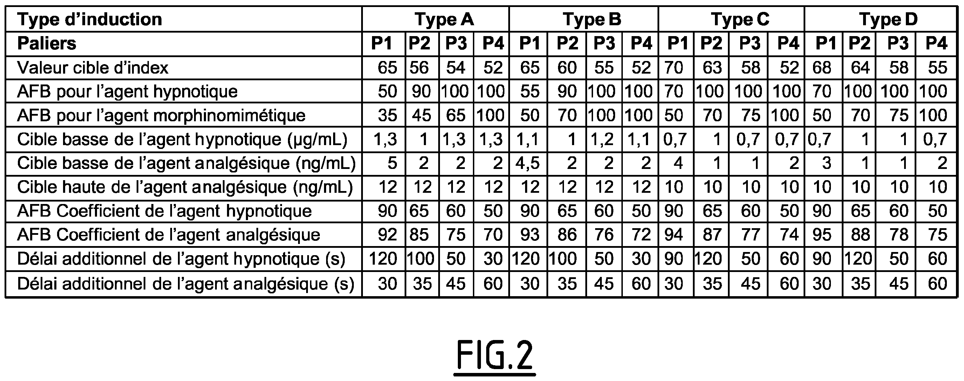

On peut résumer dans le tableau de la Figure 2 les étapes de l'induction pour un agent hypnotique tel que le Propofol et pour un agent morphinomimétique tel que le Rémifentanil.

De manière générale :

- si l'Index Error est négatif alors on diminue la concentration de façon proportionnelle par l'AFB,
- si l'Index Error est compris entre 0 et 10 avec un index nominal de 80, alors on ne modifie pas la concentration et on attend les délais comme précédemment, et
- si l'Index Error est supérieur à 10, alors on augmente la concentration des deux agents.

Le deuxième palier est quant à lui guidé avec un index nominal de 70.

Le troisième palier peut être établi avec un index nominal de 60 et des règles identiques aux autres paliers pour attendre l'expiration des délais prédéterminés.

En fait un délai supplémentaire peut être rajouté au temps calculé par le modèle. Ce délai supplémentaire est en fonction du type d'induction.

A la fin de la phase d'induction qui se définit comme étant la période entre le début de l'administration des agents anesthésiques et le maintien du signal de profondeur d'anesthésie au moins 30 secondes sous une valeur par exemple égale à 60, le contrôleur CLASS bascule en phase d'entretien. La phase d'entretien peut se décrire d'après l'observation du signal entrant qui comprend :

- l'index de qualité du signal,
- la valeur absolue de l'index enregistrée par exemple toutes les 5 secondes,
- la valeur de l'EMG ou la RE,
- la présence et la quantification de BSR,
- la pente de la variation de l'index, et
- la mesure et le calcul à partir de l'index, en utilisant des fenêtres temporelles successives de :

  • l'évolution des oscillations en fréquence et en amplitude (comme décrit précédemment),
  • le pourcentage de temps compris dans l'intervalle 40-60,
  • le pourcentage de temps des valeurs inférieures à 45, et
  • le pourcentage de temps des valeurs supérieures à 55.

De plus on prend également en compte :

- l'évolution des cibles des deux agents anesthésiques par fenêtres temporelles successives, et
- le ratio entre la valeur cible courante de chaque agent et la valeur maximum autorisée.

De même, le temps nécessaire pour atteindre l'équilibre de la concentration cible choisie et le temps continu passé à limite inférieure de l'un ou de l'autre des agents peuvent également être enregistrés.

En effet, des moyens d'établissement de valeurs limites hautes et basses de concentration des agents peuvent être définis, ces moyens étant par exemple des moyens d'entrée manuelle par l'opérateur des valeurs limites hautes et basses.

Ainsi par exemple, lors de l'utilisation en boucle fermée, ces valeurs minimales et maximales sont par exemple de 1,3 et 5 $\mu$g/mL pour l'agent hypnotique tel que le Propofol et de 3 et 15 ng/mL pour l'agent morphinomimétique tel que le Rémifentanil. Ces valeurs dépendent elles aussi du type d'induction. Elles sont plus petites en allant du type A vers le type D :

- de 1,3 à 0,7 $\mu$g/mL de limite inférieure pour l'agent hypnotique tel que le Propofol, et de 3 à 2 ng/mL de limite inférieure pour l'agent morphinomimétique tel que le Rémifentanil, et
- de 8 à 12 ng/mL de limite supérieure pour l'agent morphinomimétique tel que le Rémifentanil, la limite supérieure pour l'agent hypnotique tel que le Propofol étant toujours à 5 $\mu$g/mL.

A tout moment l'utilisateur peut décider de modifier ces valeurs limites, et à tout moment durant l'utilisation du contrôleur CLASS, sont enregistrés le temps passé à la limite inférieure de l'un ou l'autre de ces agents, l'évolution des concentrations des deux agents par fenêtres temporelles successives et le ratio entre la valeur cible courante de chaque agent et la valeur maximale autorisée.

Le traitement des oscillations se fait sur une période de quatre minutes en mesurant la fréquence et l'amplitude des oscillations du BIS, une oscillation étant une suite de deux variations de sens opposés du BIS sur une période de cinq secondes (croissance puis décroissance ou l'inverse).

Dans le cas d'oscillations de grande amplitude, c'est-à-dire où l'amplitude d'oscillation est supérieure à un premier seuil, une augmentation forfaitaire de la concentration d'agent hypnotique est déclenchée à la troisième oscillation. L'importance de cette augmentation est indexée à la cible courante de l'agent. Cette augmentation est d'autant plus faible que la valeur cible d'agent hypnotique est élevée. Cette variation de la concentration d'agent hypnotique est conditionnée à l'absence de BSR et à un nombre faible de valeurs d'index en dessous de l'intervalle de tolérance. Son déclenchement bloque celui de la rétroaction.

Quand les oscillations sont de faible amplitude, on définit trois seuils déclenchant des actions différentes s'ils sont atteints dans la fenêtre temporelle des quatre minutes. Le traitement des oscillations consiste alors à la détermination de l'agent sur lequel le système agit par la comparaison entre la valeur cible courante et la valeur cible maximum autorisée au moment de la décision pour chaque agent. Cette attitude vise à éviter un déséquilibre entre les deux principaux agents d'anesthésie dont on sait que l'action est synergique. Dans ce cas, on agira alors préférentiellement sur l'agent pour lequel le ratio valeur cible courante / valeur cible maximum est le plus faible :

- si c'est l'agent morphinomimétique :

- au premier seuil atteint par l'amplitude de l'oscillation, la concentration d'agent morphinomimétique ne peut plus diminuer,
- au deuxième seuil atteint, l'AFB du morphinomimétique est diminué pour accentuer la réaction suivante, et
- au troisième seuil, une augmentation forfaitaire est décidée immédiatement.

- si c'est l'agent hypnotique :

- au premier seuil atteint, la concentration d'agent hypnotique ne peut plus diminuer,
- au deuxième seuil atteint, l'AFB de l'hypnotique est diminué pour accentuer la réaction suivante, et
- au troisième seuil, une augmentation forfaitaire est décidée immédiatement. Dans une fenêtre de quatre minutes, il ne peut y avoir plus de trois déclenchements de cette fonction.

L'amplitude de la correction décroît du premier au troisième seuil de déclenchement. Cette fonction n'est déclenchée que si les BSR ne sont pas trop élevés.

Le contrôleur comporte alors des moyens de calcul adaptés pour calculer l'écart entre la valeur courante du signal de profondeur d'anesthésie et la valeur cible, et pour comparer cet écart à des valeurs de seuil prédéterminées pour, lorsque la valeur courante du signal de profondeur d'anesthésie est au dessus de la valeur cible :

- lorsque l'écart est inférieur à un premier seuil, agir prioritairement sur la concentration d'agent morphinomimétique, en l'augmentant,
- lorsque l'écart est supérieur au premier seuil et inférieur à un second seuil, augmenter la concentration d'agent morphinomimétique et augmenter celle de l'agent hypnotique dans une faible proportion, et
- lorsque l'écart est supérieur au second seuil, augmenter la concentration d'agent morphinomimétique et celle de l'agent hypnotique dans une plus forte proportion,

et ce pour ramener la valeur du signal de profondeur d'anesthésie dans la plage prédéterminée.

De même, lorsque la valeur courante du signal de profondeur d'anesthésie est en dessous de la valeur cible :

- lorsque l'écart est inférieur à un quatrième seuil, le contrôleur agit prioritairement sur la concentration d'agent morphinomimétique en la réduisant,
- lorsque l'écart est inférieur au quatrième seuil et supérieur à un cinquième seuil en réduisant la concentration d'agent morphinomimétique et en diminuant celle de l'agent hypnotique dans une faible proportion, et
- lorsque l'écart est inférieur au cinquième seuil, en réduisant la concentration d'agent morphinomimétique et celle de l'agent hypnotique dans une plus forte proportion,

et ce pour ramener la valeur du signal de profondeur d'anesthésie dans la plage prédéterminée.

On conçoit alors que différentes actions peuvent être engendrées par le contrôleur CLASS, à savoir :

- augmenter proportionnellement la valeur cible de l'un ou l'autre des agents,
- diminuer proportionnellement la valeur cible de l'un ou l'autre des agents,
- augmenter le délai d'attente après une modification de la valeur cible,
- diminuer le délai d'attente après une modification de la valeur cible,
- modifier la hiérarchie des actions en cours,
- augmenter la valeur de l'index nominal,
- diminuer la valeur de l'index nominal,
- bloquer la décroissance de la concentration d'agent hypnotique tel que le Propofol,
- bloquer la décroissance de la concentration d'agent morphinomimétique tel que le Rémifentanil,
- augmenter forfaitairement la concentration d'agent hypnotique tel que le Propofol,
- augmenter forfaitairement la concentration d'agent morphinomimétique tel que le Rémifentanil,
- déplacer les bornes de l'intervalle de tolérance de valeurs de l'index,
- amplifier la correction par rapport aux calculs de base, et
- modifier temporairement la limite inférieure par exemple de l'agent morphinomimétique ou hypnotique.

Par ailleurs, des sécurités sont également mises en place. Par exemple, des moyens de désactivation de l'un et/ou l'autre des moyens de calcul des ordres de pilotage des moyens d'injection peuvent être envisagés, afin de permettre à l'opérateur une reprise manuelle de la commande des moyens d'injection des agents.

De même, des moyens de maintien des ordres de commande à leur dernière valeur avant une perte du signal d'activité électrocorticale peuvent être envisagés, ces moyens étant par exemple associés à des moyens d'alerte sonore et/ou visuelle de l'opérateur en cas de perte de ce signal.

A tout moment, l'utilisateur peut administrer les agents d'anesthésie en mode manuel.

Ces différents moyens peuvent être mis en œuvre par des programmes logiciels intégrés dans le calculateur 5.

On conçoit alors qu'un tel système permet d'administrer simultanément en boucle fermée un agent hypnotique tel que le Propofol et un agent morphinomimétique tel que le Rémifentanil, et ce de façon asservie à partir d'un seul signal d'activité électrocorticale du patient.

Un contrôleur CLASS permet l'induction et l'entretien de l'anesthésie générale, la sédation de la période post-opératoire et la sédation pour les patients en réanimation.

Il utilise comme signal entrant l'activité électrocorticale fournie par un moniteur par exemple d'index bispectral ou d'entropie, ce qui permet de mesurer la profondeur de l'anesthésie.

## Revendications

1. Système de pilotage de moyens d'injection d'agents d'anesthésie ou de sédation en mode d'anesthésie ou de sédation intraveineuse à objectif de concentration ou de débit massique, à un patient (1), en vue de l'induction et de l'entretien de cette anesthésie ou de cette sédation, les moyens d'injection d'agents d'anesthésie comprenant des premiers moyens (7) d'injection d'un agent hypnotique et des seconds moyens (8) d'injection d'un agent morphinomimétique, comportant :

   - des moyens (2) d'acquisition d'un signal représentatif de l'activité électrocorticale du patient (1),
   - des moyens (3) d'analyse de ce signal pour en tirer un signal de profondeur d'anesthésie, et
   - des moyens (4, 5, 6) de suivi de la valeur et de l'évolution dans le temps de ce signal de profondeur d'anesthésie ;

   ce que les moyens (4, 5, 6) de suivi de la valeur et de l'évolution dans le temps du signal de profondeur d'anesthésie sont associés à des moyens (4, 5, 6) de calcul d'ordres de pilotage des premiers et des seconds moyens d'injection des agents hypnotique et morphinomimétique, afin :

   - d'amener automatiquement le signal de profondeur d'anesthésie au-dessous d'une valeur cible pendant au moins 30 secondes lors d'une phase d'induction de l'anesthésie, la phase d'induction de l'anesthésie étant la période entre le début de l'administration des agents et le maintien du signal de profondeur d'anesthésie au moins 30 secondes sous la valeur cible, puis
   - de réguler automatiquement celui-ci en boucle fermée dans une plage prédéterminée lors d'une phase d'entretien de cette anesthésie ;

   et en ce que les moyens de calcul des ordres de pilotage des moyens d'injection comprennent :

   - des moyens d'entrée par un opérateur d'un type d'induction d'anesthésie choisi parmi un ensemble de types d'induction se différenciant par la concentration initiale d'agent hypnotique, comprenant des moyens d'établissement de la concentration initiale d'agent hypnotique, à laquelle est associée une concentration initiale d'agent morphinomimétique prédéterminée selon le type d'induction sélectionné, et
   - des moyens d'établissement de valeurs de signal de profondeur d'anesthésie intermédiaires pour définir des paliers lors de la phase d'induction de l'anesthésie.

2. Système de pilotage selon la revendication 1, **caractérisé en ce que** les moyens d'établissement sont adaptés pour établir trois valeurs intermédiaires de paliers d'anesthésie.

3. Système de pilotage selon l'une quelconque des revendications précédentes, **caractérisé en ce que** les premiers moyens (7) d'injection de l'agent hypnotique reçoivent des ordres de pilotage à une première fréquence et **en ce que** les seconds moyens (8) d'injection de l'agent morphinomimétique reçoivent des ordres de pilotage à une seconde fréquence différente de la première.

4. Système de pilotage selon la revendication 3, **caractérisé en ce que** la seconde fréquence est supérieure à la première fréquence.

5. Système de pilotage selon l'une quelconque des revendications précédentes, **caractérisé en ce qu'**il comporte des moyens d'entrée par un opérateur de la valeur cible de signal de profondeur d'anesthésie et des moyens d'établissement d'une valeur cible par défaut.

6. Système de pilotage selon l'une quelconque des revendications précédentes, **caractérisé en ce que** les moyens de calcul comportent des moyens de limitation et/ou d'inhibition d'ordres de pilotage en sens opposés des premiers et seconds moyens d'injection.

7. Système de pilotage selon l'une quelconque des revendications précédentes, **caractérisé en ce que** les moyens de calcul sont adaptés pour augmenter la concentration d'agent hypnotique après un nombre prédéterminé de modifications de la concentration d'agent morphinomimétique pour ramener le signal de profondeur d'anesthésie dans la plage prédéterminée autour de la valeur cible.

8. Système de pilotage selon la revendication 7, **caractérisé en ce que** le nombre prédéterminé de modifications est égal à trois.

9. Système de pilotage selon l'une quelconque des revendications précédentes, **caractérisé en ce que** les moyens de calcul sont adaptés pour calculer l'écart entre la valeur courante du signal de profondeur d'anesthésie et la valeur cible, et pour comparer cet écart à des valeurs de seuil prédéterminées pour, lorsque la valeur courante du signal de profondeur d'anesthésie est au-dessus de la valeur cible :

- lorsque l'écart est inférieur à un premier seuil, agir prioritairement sur la concentration d'agent morphinomimétique, en l'augmentant,
- lorsque l'écart est supérieur au premier seuil et inférieur à un second seuil, augmenter la concentration d'agent morphinomimétique et augmenter l'agent hypnotique dans une faible proportion, et
- lorsque l'écart est supérieur au second seuil, augmenter la concentration d'agent morphinomimétique et celle de l'agent hypnotique dans une plus forte proportion,

pour ramener la valeur du signal de profondeur d'anesthésie dans la plage prédéterminée.

10. Système de pilotage selon l'une quelconque des revendications précédentes, **caractérisé en ce que** les moyens de calcul sont adaptés pour calculer l'écart entre la valeur courante du signal de profondeur d'anesthésie et la valeur cible, et pour comparer cet écart à des valeurs de seuil prédéterminées si le signal est inférieur à la valeur cible pour, lorsque la valeur courante du signal de profondeur d'anesthésie est en-dessous de la valeur cible :

- lorsque l'écart est inférieur à un troisième seuil, agir prioritairement sur la concentration d'agent morphinomimétique en la réduisant,
- lorsque l'écart est inférieur au troisième seuil et supérieur à un cinquième seuil, réduire la concentration d'agent morphinomimétique et diminuer celle de l'agent hypnotique dans une faible proportion, et
- lorsque l'écart est inférieur au quatrième seuil, réduire la concentration d'agent morphinomimétique et celle de l'agent hypnotique dans une plus forte proportion,

pour ramener la valeur du signal de profondeur d'anesthésie dans la plage prédéterminée.

11. Système de pilotage selon l'une quelconque des revendications précédentes, **caractérisé en ce qu'**il comporte des moyens d'établissement de valeurs limites hautes et basses de concentrations des agents hypnotique et morphinomimétique.

12. Système de pilotage selon la revendication 11, **caractérisé en ce qu'**il comporte des moyens d'entrée manuelle par l'opérateur des valeurs limites hautes et basses de concentrations des agents hypnotique et morphinomimétique.

13. Système de pilotage selon l'une quelconque des revendications précédentes, **caractérisé en ce qu'**il comporte des moyens de désactivation de l'un et/ou l'autre des moyens de calcul des ordres de pilotage des moyens d'injection, afin de permettre à l'opérateur une reprise manuelle de la commande des moyens d'injection des agents.

14. Système de pilotage selon l'une quelconque des revendications précédentes, **caractérisé en ce qu'**il comporte des moyens de maintien des ordres de commande à leur dernière valeur avant une perte du signal d'activité électrocorticale.

**15.** Système de pilotage selon la revendication 14, **caractérisé en ce qu'**il comporte des moyens d'alerte sonore et/ou visuelle de l'opérateur en cas de perte du signal d'activité électrocorticale.

**16.** Système de pilotage selon l'une quelconque des revendications précédentes, **caractérisé en ce que** les moyens d'injection comprennent des moyens motorisés associés à des contrôleurs de pilotage de leur fonctionnement.

**Patentansprüche**

**1.** System zum Steuern von Mitteln zur Injektion in einen Patienten (1) von Anästhesie- oder Sedierungsmitteln im Anästhesie- oder intravenöse Sedierungsmodus mit Ziel der Konzentration oder des Massenflusses in Hinblick auf die Einleitung und die Aufrechterhaltung dieser Anästhesie oder dieser Sedierung, wobei die Mittel zur Injektion von Anästhesiemitteln erste Mittel (7) zur Injektion eines hypnotischen Mittels und zweite Mittel (8) zur Injektion eines Morphinomimetikums umfassen, enthaltend:

- Mittel (2) zum Beschaffen eines Signals, das für die electrocortikale Aktivität des Patienten (1) repräsentativ ist,
- Mittel (3) zum Analysieren dieses Signals, um daraus ein Signal der Anästhesietiefe herauszuziehen, und
- Mittel (4, 5, 6) zum Verfolgen des Wertes und des zeitlichen Verlaufs dieses Signals der Anästhesietiefe;

wobei die Mittel (4, 5, 6) zum Verfolgen des Wertes und des zeitlichen Verlaufs Mitteln (4, 5, 6) zum Berechnen von Steuerbefehlen der ersten und zweiten Mittel zur Injektion des hypnotischen Mittels und des Morphinomimetikums, um:

- automatisch das Signal der Anästhesietiefe unter einen Zielwert während mindestens 30 Sekunden bei einer Phase der Einleitung der Anästhesie zu bringen, wobei die Phase der Einleitung der Anästhesie der Zeitraum zwischen dem Beginn der Verabreichung der Mittel und des Haltens des Signals der Anästhesietiefe über mindestens 30 Sekunden unter dem Zielwert ist, dann
- automatisch diesen in einem geschlossenen Kreis in einem vorbestimmten Bereich während der Aufrechterhaltung dieser Anästhesie zu regeln;

und dass die Mittel zum Berechnen der Steuerbefehle der Mittel zur Injektion umfassen:

- Mittel zum Eingeben durch eine Bedienperson eines Typs der Einleitung der Anästhesie, ausgewählt aus einer Gesamtheit von sich durch die Anfangskonzentration des hypnotischen Mittels unterscheidenden Einleitungstypen, die Mittel zum Festlegen der Anfangskonzentration des hypnotischen Mittels umfassen, der eine gemäß dem Typ der ausgewählten Einleitung vorbestimmte Anfangskonzentration des Morphinomimetikums zugeordnet ist, und
- Mittel zum Festlegen von Zwischenwerten des Signals der Anästhesietiefe, um Abstufungen während der Phase der Einleitung der Anästhesie zu definieren.

**2.** Steuersystem nach Anspruch 1, **dadurch gekennzeichnet, dass** die Mittel zum Festlegen angepasst sind, drei Zwischenwerte der Abstufungen der Anästhesie festzulegen.

**3.** Steuersystem nach einem beliebigen der vorhergehenden Ansprüche, **dadurch gekennzeichnet, dass** die ersten Mittel (7) zur Injektion des hypnotischen Mittels Steuerbefehle bei einer ersten Frequenz empfangen und dass die zweiten Mittel (8) zur Injektion des Morphinomimetikums Steuerbefehle bei einer zweiten zu der ersten unterschiedlichen Frequenz empfangen.

**4.** Steuersystem nach Anspruch 3, **dadurch gekennzeichnet, dass** die zweite Frequenz über der ersten Frequenz liegt.

**5.** Steuersystem nach einem beliebigen der vorhergehenden Ansprüche, **dadurch gekennzeichnet, dass** es Mittel zum Eingeben des Zielwerts des Signals der Anästhesietiefe durch eine Bedienperson und Mittel zum Festlegen eines Standardzielwertes aufweist.

**6.** Steuersystem nach einem beliebigen der vorhergehenden Ansprüche, **dadurch gekennzeichnet dass** die Berechnungsmittel Mittel zum Begrenzen und/oder zum Sperren von Steuerbefehlen in entgegengesetzter Richtung der ersten und zweiten Mittel zur Injektion aufweisen.

**7.** Steuersystem nach einem beliebigen der vorhergehenden Ansprüche, **dadurch gekennzeichnet, dass** die Berechnungsmittel angepasst sind, die Konzentrationen des hypnotischen Mittels nach einer vorbestimmten Anzahl von Modifikationen der Konzentration des Morphinomimetikums zu erhöhen, um das Signal der Anästhesietiefe in dem vorbestimmten Bereich um den Zielwert herum zurückzubringen.

**8.** Steuersystem nach Anspruch 7, **dadurch gekennzeichnet, dass** die vorbestimmte Anzahl von Modifikationen gleich drei ist.

**9.** Steuersystem nach einem beliebigen der vorhergehenden Ansprüche, **dadurch gekennzeichnet, dass** die Berechnungsmittel angepasst sind, den Abstand zwischen dem aktuellen Wert des Signals der Anästhesietiefe und dem Zielwert zu berechnen und diesen Abstand mit vorbestimmten Schwellenwerten zu vergleichen, um, wenn der aktuelle Wert des Signals der Anästhesietiefe über dem Zielwert liegt:

- vorrangig auf die Konzentration des Morphinomimetikums zu wirken, indem sie erhöht wird, wenn der Abstand kleiner als eine erste Schwelle ist,
- die Konzentration des Morphinomimetikums und die des hypnotischen Mittels in einem geringeren Anteil zu erhöhen, wenn der Abstand größer als die erste Schwelle und kleiner als eine zweite Schwelle ist, und
- die Konzentration des Morphinomimetikums und die des hypnotischen Mittels in einem größeren Anteil zu erhöhen, wenn der Abstand größer als die zweite Schwelle ist,
um den Wert des Signals der Anästhesietiefe in den vorbestimmten Bereich zurückzubringen.

**10.** Steuersystem nach einem beliebigen der vorhergehenden Ansprüche, **dadurch gekennzeichnet, dass** die Berechnungsmittel angepasst sind, den Abstand zwischen dem aktuellen Wert des Signals der Anästhesietiefe und dem Zielwert zu berechnen und diesen Abstand mit vorbestimmten Schwellenwerten zu vergleichen, um, wenn der aktuelle Wert des Signals der Anästhesietiefe unter dem Zielwert liegt:

- vorrangig auf die Konzentration des Morphinomimetikums zu wirken, indem sie verringert wird, wenn der Abstand kleiner als eine dritte Schwelle ist, und
- die Konzentration des Morphinomimetikums zu senken und die des hypnotischen Mittels in einem geringeren Anteil zu verringern, wenn der Abstand kleiner als die dritte Schwelle und größer als eine fünfte Schwelle ist,
- die Konzentration des Morphinomimetikums und die des hypnotischen Mittels in einem größeren Anteil zu verringern, wenn der Abstand kleiner als die vierte Schwelle ist,
um den Wert des Signals der Anästhesietiefe in den vorbestimmten Bereich zurückzubringen.

**11.** Steuersystem nach einem beliebigen der vorhergehenden Ansprüche, **dadurch gekennzeichnet, dass** es Mittel zum Festlegen von oberen und unteren Grenzwerten der Konzentrationen des hypnotischen Mittels und des Morphinomimetikums aufweist.

**12.** Steuersystem nach Anspruch 11, **dadurch gekennzeichnet, dass** es Mittel zur manuellen Eingabe von oberen und unteren Grenzwerten der Konzentrationen des hypnotischen Mittels und des Morphinomimetikums durch die Bedienperson aufweist.

**13.** Steuersystem nach einem beliebigen der vorhergehenden Ansprüche, **dadurch gekennzeichnet, dass** es Mittel zur Deaktivierung des einen und/oder des anderen der Berechnungsmittel der Steuerbefehle der Mittel zur Injektion aufweist, um der Bedienperson eine manuelle Wiederholung der Steuerung der Mittel zur Injektion der Mittel aufweist.

**14.** Steuersystem nach einem beliebigen der vorhergehenden Ansprüche, **dadurch gekennzeichnet, dass** es Mittel zum Halten der Steuerbefehle auf ihrem letzten Wert vor einem Verlust des Signals der electrocortikalen Aktivität aufweist.

**15.** Steuersystem nach Anspruch 14, **dadurch gekennzeichnet, dass** es Mittel eines akustischen und/oder visuellen Alarms für die Bedienperson im Falle eines Verlusts des Signals der electrocortikalen Aktivität aufweist.

**16.** Steuersystem nach einem beliebigen der vorhergehenden Ansprüche, **dadurch gekennzeichnet, dass** die Mittel zur Injektion motorisierte Mittel umfassen, die Reglern zur Steuerung ihrer Funktionsweise zugeordnet sind.

**Claims**

1. A system for controlling means for injecting anesthesia or sedation agents in an intravenous anesthesia or sedation mode with a concentration or mass flow rate target, into a patient (1), for inducing and maintaining this anesthesia or this sedation, the means for injecting anesthesia agents comprising first means (7) for injecting a hypnotic agent and second means (8) for injecting a morphinomimetic agent, comprising:

   - means (2) for acquiring a signal representative of the electrocortical activity of the patient (1),
   - means (3) for analyzing this signal in order to extract therefrom an anesthetic depth signal,
   - means (4, 5, 6) for tracking the value and time-dependent evolution of this anesthetic depth signal;

   the means (4, 5, 6) for tracking the value and time-dependent evolution of this anesthetic depth signal being associated with means (4, 5, 6) for computing control signals for controlling the first and the second means for injecting the hypnotic and morphinomimetic agent, in order to:

   - automatically bring the anesthetic depth signal below a target value for at least 30 seconds during a phase for inducing the anesthesia, the phase for inducing the anesthesia being the period between the beginning of the administration of the agents and the maintaining of the anesthetic depth signal at least 30 seconds below the target value, then
   - automatically regulating this one in a closed loop in a predetermined range during a phase for maintaining the anesthesia ;

   and in that the means for computing commands for controlling the injection means comprise :

   - means for inputting by an operator a type of anesthesia induction selected from a set of induction types which differ by the initial concentration of hypnotic agent, comprising means for determining the initial concentration of hypnotic agent, with which is associated a predetermined initial concentration of morphinomimetic agent depending on the selected type of induction, and
   - means for determining intermediate target values for the anesthetic depth signal in order to define plateaus during the induction phase.

2. The control system according to claim 1, **characterized in that** the establishment means are adapted in order to establish three intermediate values of anesthesia plateaus.

3. The control system according to any one of the preceding claims, **characterized in that** the first means (7) for injecting the hypnotic agent receive control signals at a first frequency and **in that** the second means (8) for injecting the morphinomimetic agent receive control signals at a second frequency different from the first frequency.

4. The control system according to claim 3, **characterized in that** the second frequency is higher than the first frequency.

5. The control system according to any one of the preceding claims, **characterized in that** it includes means for inputting by an operator the target value of the anesthetic depth signal and means for determining a target value by default.

6. The control system according to any one of the preceding claims, **characterized in that** the computing means include means for limiting and/or inhibiting control signals in opposite directions of the first and second injection means.

7. The control system according to any one of the preceding claims, **characterized in that** the computing means are adapted for increasing the concentration of hypnotic agent after a predetermined number of modifications of the morphinomimetic agent concentration for bringing back the anesthetic depth signal into the predetermined range around the target value.

8. The control system according to claim 7, **characterized in that** the predetermined number of modifications is equal to three.

9. The control system according to any one of the preceding claims, **characterized in that** the computing means are adapted for computing the difference between the current value of the anesthetic depth signal and the target value, and for comparing this difference with predetermined threshold values in order to, when the current value of the anesthetic depth signal is above the target value:

- when the difference is less than a first threshold, act with priority on the concentration of morphinomimetic agent, by increasing it,
- when the difference is greater than the first threshold and less than a second threshold, increase the concentration of morphinomimetic agent and increase the hypnotic agent in a small proportion, and
- when the difference is greater than the second threshold, increase the concentration of the morphinomimetic agent and that of the hypnotic agent in a larger proportion,

in order to bring the value of the anesthesia depth signal back into the predetermined range.

10. The control system according to any one of the preceding claims, **characterized in that** the computing means are adapted for computing the difference between the current value of the anesthetic depth signal and the target signal, and for comparing this difference with predetermined threshold values if the signal is less than the target value in order to, when the current value of the anesthetic depth signal is below the target value:

- when the difference is less than a third threshold, act with priority on the concentration of morphinomimetic agent by reducing it,
- when the difference is less than the third threshold and above a fifth threshold, reduce the concentration of morphinomimetic agent and reduce that of the hypnotic agent in a small proportion, and
- when the difference is less than the fourth threshold, reduce the concentration of morphinomimetic agent and that of the hypnotic agent in a larger proportion,

in order to bring the value of the anesthetic depth signal back into the predetermined range.

11. The control system according to any one of the preceding claims, **characterized in that** it includes means for determining high and low limiting values of the concentrations of the hypnotic and morphinomimetic agents.

12. The control system according to claim 11, **characterized in that** it includes means for the operator to manually input high and low limiting values of the concentrations of the hypnotic and morphinomimetic agents.

13. The control system according to any one of the preceding claims, **characterized in that** it includes means for disabling either one or both of the means for computing control signals for controlling the injection means, so as to allow the operator to take over manually the control of the means for injecting the agents.

14. The control system according to any one of the preceding claims, **characterized in that** it includes means for maintaining control signals at their last value before loss of the electrocortical activity signal.

15. The control system according to claim 14, characterized that it includes sound and/or visual means for alerting the operator in the case of loss of the electrocortical activity signal.

16. The control system according to any one of the preceding claims, **characterized in that** the injection means comprise motorized means associated with controllers for controlling their operation.

**FIG.1**

| Type d'induction | Type A | | | | Type B | | | | Type C | | | | Type D | | | |
|---|---|---|---|---|---|---|---|---|---|---|---|---|---|---|---|---|
| Paliers | P1 | P2 | P3 | P4 | P1 | P2 | P3 | P4 | P1 | P2 | P3 | P4 | P1 | P2 | P3 | P4 |
| Valeur cible d'index | 65 | 56 | 54 | 52 | 65 | 60 | 55 | 52 | 70 | 63 | 58 | 52 | 68 | 64 | 58 | 55 |
| AFB pour l'agent hypnotique | 50 | 90 | 100 | 100 | 55 | 90 | 100 | 100 | 70 | 100 | 100 | 100 | 70 | 100 | 100 | 100 |
| AFB pour l'agent morphinomimétique | 35 | 45 | 65 | 100 | 50 | 70 | 100 | 100 | 50 | 70 | 75 | 100 | 50 | 70 | 75 | 100 |
| Cible basse de l'agent hypnotique (µg/mL) | 1,3 | 1 | 1,3 | 1,3 | 1,1 | 1 | 1,2 | 1,1 | 0,7 | 1 | 0,7 | 0,7 | 0,7 | 1 | 1 | 0,7 |
| Cible basse de l'agent analgésique (ng/mL) | 5 | 2 | 2 | 2 | 4,5 | 2 | 2 | 2 | 4 | 1 | 1 | 2 | 3 | 1 | 1 | 2 |
| Cible haute de l'agent analgésique (ng/mL) | 12 | 12 | 12 | 12 | 12 | 12 | 12 | 12 | 10 | 10 | 10 | 10 | 10 | 10 | 10 | 10 |
| AFB Coefficient de l'agent hypnotique | 90 | 65 | 60 | 50 | 90 | 65 | 60 | 50 | 90 | 65 | 60 | 50 | 90 | 65 | 60 | 50 |
| AFB Coefficient de l'agent analgésique | 92 | 85 | 75 | 70 | 93 | 86 | 76 | 72 | 94 | 87 | 77 | 74 | 95 | 88 | 78 | 75 |
| Délai additionnel de l'agent hypnotique (s) | 120 | 100 | 50 | 30 | 120 | 100 | 50 | 30 | 90 | 120 | 50 | 60 | 90 | 120 | 50 | 60 |
| Délai additionnel de l'agent analgésique (s) | 30 | 35 | 45 | 60 | 30 | 35 | 45 | 60 | 30 | 35 | 45 | 60 | 30 | 35 | 45 | 60 |

## FIG.2

EP 2 389 208 B1

**EP 2 389 208 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 7220240 B
- US 6631291 B
- US 2004079372 A
- EP 1547631 A